# EUROPEAN PATENT APPLICATION

(11) **EP 4 026 837 A1**
(43) Date of publication of application: **13.07.2022**
(21) Application number: 21150632.4
(22) Date of filing: 08.01.2021
(51) Int. Cl.: C07D 493/04

(54) **PROCESS FOR PRODUCING 2,5-DIAMINO-2,5-DIDEOXY-1,4:3,6-DIANHYDROHEXITOL**

(71) Applicant: Covestro Deutschland AG, 51373 Leverkusen (DE)
(72) Inventor: RUIJING, Guo, 201208 Shanghai (CN); PIRES, Raul, 50670 Köln (DE); BEUCK, Saskia, 51375 Leverkusen (DE); LATORRE MARTINEZ, Irene Cristina, 51373 Leverkusen (DE); PAN, Xiangcheng, 200438 Shanghai (CN); WANG, Qianyi, 200438 Shanghai (CN); YANG, Shicheng, 200438 Shanghai (CN); JIANG, Yuan, 200438 Shanghai (CN)
(74) Representative: Levpat

(57) **Abstract**

The invention relates to a process for the synthesis of chiral diamines based on renewable resources. More specifically the invention provides a process for synthesis of diaminoisoidide, diaminoisosorbide, and diaminoisomannide, more specifically 2,5-diamino-2,5-dideoxy-1,4:3,6-dianhydrohexitols.

## Description

The invention relates to a process for the synthesis of chiral diamines based on renewable resources. More specifically the invention provides a process for synthesis of diaminoisoidide, diaminoisosorbide, and diaminoisomannide, more specifically 2,5-diamino-2,5-dideoxy-1,4:3,6-dianhydrohexitols.

There are growing interests on bio-based chemicals in the industry following increasing awareness and demands of renewable materials to maintain a sustainable economic growth.

As an important industrial raw material, dianhydrohexitol diamines are getting more and more attention. Synthetic routes have been studied and disclosed such as routs involving conversion of the isohexide to the corresponding bismesylate, followed by nucleophilic substitution with azide.

Renewable Rigid Diamines: Efficient, Stereospecific, Synthesis of High Purity Isohexide Diamines (Shanmugam Thiyagarajan, Linda Gootjes, Willem Vogelzang, Jacco van Haveren, Martin Lutz, and Daan S. van Es, ChemSusChem 2011, 4, 1823 - 1829 _ 2011 Wiley-VCH Verlag GmbH& Co. KGaA, Weinheim) disclosed a three-step strategy for synthesizing rigid, chiral isohexide diamines derived from 1,4:3,6-dianhydrohexitols. These biobased chiral building blocks are presently the subject of several investigations (in our and several other groups) because of their application in high-performance biobased polymers, such as polyamides and polyurethanes. Among the three possible stereo-isomers, diaminoisoidide, diaminoisosorbide, and diaminoisomannide can be synthesized from isomannide, isosorbide and isoidide respectively in high yield with absolute stereo control. Furthermore, by using this methodology dideoxy-amino isomannide-a tricyclic adduct-was obtained starting from isoidide in high yield.

Isomannide-Derived Chiral Rigid Fully Biobased Polybenzoxazines (Nagarjuna Amarnath, Swapnil Shukla, and Bimlesh Lochab, ACS Sustainable Chem. Eng. 2019, 7, 18700-18710) published a novel, semicrystalline polyamides and co-(polyamides) that were synthesized from biobased sebacic acid (SA), 2,5-diamino-2,5-dideoxy-1,4;3,6-dianhydroiditol (diaminoisoidide, DAII) as well as from 1,4-diaminobutane (DAB), also known as putrescine in nature. Low molecular weight polyamides were obtained by melt polycondensation of the salts based on these monomers or by interfacial polycondensation. In order to increase their molecular weights, the polyamide prepolymers were submitted to a solid-state polymerization (SSP) process. The chemical structure of the polymers was confirmed by 2D NMR correlation spectra (COSY), heteronuclear multiple-bond correlation spectra (HMBC) and by FT-IR spectroscopy. In the present work, FT-IR and X-ray techniques were used as a tool for the investigation of the crystal structure of the polymers after SSP. The X-ray diffractograms of the polyamides point to crystals containing both 4.10- and DAII.10-based repeat units. Because of the presence of diaminoisoidide residues the synthesized fully renewable products exhibit tunable polarities and melting points.

US2017/0081338A1 provided novel heteroaryl compounds, pharmaceutical acceptable salts and formulations thereof. They are useful in preventing, managing, treating or lessening the severity of a protein kinase-mediated disease. The invention also provides pharmaceutically acceptable compositions comprising such compounds and methods of using the compositions in the treatment of protein kinase-mediated disease.

It is tried and known to provide processes in providing diaminoisoidide such as above-mentioned approaches. However, those approaches are either at very small amount of production, or resulting in low yield, holding the development or commercialization of the synthesis of diaminoisoidide via bio-based raw materials.

There is therefore a need for a process for preparing diaminoisoidide with larger scale and higher yields.

**One aspect of the present invention** is concerned specifically on an effective approach in the synthesis of diaminoisoidide.

Taking account of above need, the present invention provides a method for producing 2,5-diamino-2,5-dideoxy-1,4:3,6-dianhydrohexitol, comprising:
a). synthesis of 1,4:3,6-dianhydro-2,5-di-O-p-tosyl-hexitol by reacting of 1,4:3,6-dianhydrohexitol, preferably isomannide, and at least a toluenesulfonyl containing compound;
b). synthesis of 2,5-diphthalimido-2,5-dideoxy-1,4:3,6-dianhydrohexitol by reacting of 1,4:3,6-dianhydro-2,5-di-O-p-tosyl-hexitol, preferably 1,4:3,6-dianhydro-2,5-di-O-p-tosyl-D-mannitol, and a phthalimide containing compound;
c). synthesis of 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride, by reacting of 2,5-diphthalimido-2,5-dideoxy-1,4:3,6-dianhydrohexitol with at least an acid and at least a chloride source, preferably HCl, more preferably a mixture of HCl and AcOH;
d). reacting of 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride, and at least an alkali, preferably NaOH, and purification, preferably recrystallization to provide the 2,5-diamino-2,5-dideoxy -1,4:3 ,6-dianhydrohexitol.

Preferably, the 2,5-diamino-2,5-dideoxy-1,4:3,6-dianhydrohexitol is selected from 2,5-diamino-2,5-dideoxy-1,4:3,6-dianhydroiditol.

Preferably, the chloride source is selected from hydrochloric acid (HCl), sodium chloride (NaCl), potassium chloride (KCl), lithium chloride (LiCl), ammonium chloride (NH4Cl), tetrabutylammonium chloride (Bu4NCl) and any other chloride salts.

Preferably, the 1,4:3,6-dianhydro-2,5-di-O-p-tosyl-hexitol is selected from 1,4:3,6-dianhydro-2,5-di-Op-tosyl-D-mannitol.

Preferably, the 2, 5-diphthalimido-2,5-dideoxy-1,4:3,6-dianhydrohexitol is selected from 2,5-diphthalimido-2,5-dideoxy-1,4:3,6-dianhydroiditol. Preferably, the 2,5-diamino-2,5-dideoxy-1,4:3,6-dianhydrohexitol has a structure of:

| | |
|---|---|
| 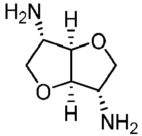 | **5a,** C2/C5 endo |
| | 2,5-diamino-2,5-dideoxy-1,4:3,6-dianhydroiditol |
| | |
| 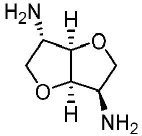 | **5b,** C2 exo/c5 endo |
| | 2,5-diamino-2,5-dideoxy-1,4:3,6-dianhydroglucidol |
| | |
| 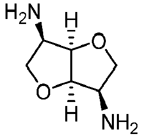 | **5c,** C2/C5 exo |
| | 2,5-diamino-2,5-dideoxy-1,4:3,6-dianhydromannitol |

and, preferably a structure of below, which is 2,5-diamino-2,5-dideoxy-1,4:3,6-dianhydroiditol:

| | |
|---|---|
| 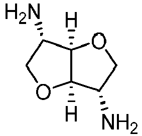 | **5a,** C2/C5 endo |
| | 2,5-diamino-2,5-dideoxy-1,4:3,6-dianhydroiditol |

Preferably, using isomannide as raw material through the method of the invention, the preferred structure of above (5a) 2,5-diamino-2,5-dideoxy-1,4:3,6-dianhydroiditol is obtained.

Preferably, the toluenesulfonyl containing compound is selected from p-toluenesulfonyl chloride, p-toluenesulfonyl hydrazide, p-toluenesulfonyl isocyanate, p-toluenesulfonyl azide, p-toluenesulfonyl cyanide, m-toluenesulfonyl chloride and o-toluenesulfonyl chloride.

Preferably, there is no cation exchange resin used in the step d), preferably no cation exchange resin is used in the method.

Optionally, there is no organic solvent applied in the purification in step b).

Optionally, a filtration is applied in step b) to get solid product from the reaction of 1,4:3,6-dianhydro-2,5-di-O-p-tosyl-hexitol, preferably 1,4:3,6-dianhydro-2,5-di-O-p-tosyl-D-mannitol and the phthalimide containing compound.

Preferably, the amount of 1,4:3,6-dianhydrohexitol, preferably isomannide in step a) is ≥300g, preferably ≥320g, and more preferably ≥380g.

Preferably, the molar yield of 1,4:3,6-dianhydro-2,5-di-O-p-tosyl-hexitol, preferably 1,4:3,6-dianhydro-2,5-di-O-p-tosyl-D-mannitol in step a) is ≥50%, preferably ≥80%, more preferably ≥90%.

Optionally, the molar yield of the 2,5-diphthalimido-2,5-dideoxy-1,4:3,6-dianhydrohexitol, preferably 2, 5-diamino-2, 5-dideoxy-1, 4: 3, 6-dianhydroiditol dihydrochloride from step b) is ≥50%, preferably ≥60%.

Preferably, the volume ratio of HCl and AcOH is 5:1-3:1, preferably 4:1 in step c).

Preferably, the phthalimide containing compound is selected from potassium phthalimide, sodium phthalimide, lithium phthalimide or any of their combination.

Optionally, the molar yield of the 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride in step c) is ≥80%, preferably ≥90%.

Preferably, the molar yield of the 2,5-diamino-2,5-dideoxy-1,4:3,6-dianhydrohexitol, preferably 2,5-diamino-2,5-dideoxy-1,4:3,6-dianhydroiditol is ≥80%, preferably ≥90%.

Organic solvent in the invention could be tetrahydrofuran, diethyl ether, dioctyl ether, chloromethyl methyl ether, diethylene glycol dimethyl ether and/or diphenyl ether. In the invention, much less organic solvent is applied comparing what is used in the prior art.

**Another aspect of the present invention** is concerned specifically on 2,5-diamino-2,5-dideoxy-1,4:3,6-dianhydrohexitol prepared by the method as described above.

Preferably, the 2,5-diamino-2,5-dideoxy-1,4:3,6-dianhydrohexitol is selected from 2,5-diamino-2,5-dideoxy-1,4:3,6-dianhydroiditol.

Preferably, the purity of the 2,5-diamino-2,5-dideoxy-1,4:3,6-dianhydroiditol is ≥98%, preferably ≥99%.

**Another aspect of the present invention** is concerned specifically use of the 2,5-diamino-2,5-dideoxy-1,4:3,6-dianhydrohexitol prepared by the method according to any of the methods as described above in producing isocyanates.

Preferably, the 2,5-diamino-2,5-dideoxy-1,4:3,6-dianhydrohexitol is selected from 2,5-diamino-2,5-dideoxy-1,4:3,6-dianhydroiditol.

Embodiments of the invention are described in detail hereinafter. These may be combined with one another as desired, unless the context unambiguously suggests anything different to the person skilled in the art. Through various embodiments, surprisingly, we find that the methods of the present invention successfully achieved that, firstly, a much larger scale of production of diaminoisoidides; secondly, there are much less use of organic solvent and therefore more environment-friendly; thirdly, no expensive cation exchange resin is used in step d) and in the method; and fourthly, more effective means are provided to produce diaminoisoidides with a much higher purity and yield.

In the method of the invention, the starting state that exists in each case is converted to the state of production under normal conditions in such a way that the problems mentioned at the outset occur to a slight extent at most, if at all, as set out in detail hereinafter.

**In step a)** of the method of the invention, it is about synthesis of 2,5-diamino-2,5-dideoxy-1,4:3,6-dianhydrohexitol, preferably 1,4:3,6-dianhydro-2,5-di-O-p-tosyl-D-mannitol by reacting of 1,4:3,6-dianhydrohexitol, preferably isomannide **(1)** and at least a toluenesulfonyl containing compound. Certain amount of 1,4:3,6-dianhydrohexitol, preferably isomannide, such as 1,4:3,6-dianhydro-D-mannitol and p-toluenesulfonyl chloride, p-toluenesulfonyl hydrazide, p-toluenesulfonyl isocyanate, p-toluenesulfonyl azide, p-toluenesulfonyl cyanide, m-toluenesulfonyl chloride, o-toluenesulfonyl chloride or p-toluenesulfonyl fluoride, preferably p-toluenesulfonyl chloride and dichloromethane, DMSO dimethyl sulfoxide, DMF Dimethylformamide, MeCN acetonitrile, THF tetrahydrofuran, ethyl acetate, acetone, nitromethane and/or propylene carbonate are added into a container e.g. round-bottom flask. Then at least a solvent, which can be selected from triethylamine, pyridine, alkaneamines including methylamine, imidazole, benzimidazole, histidine, guanidine, phosphazene bases and/or any of their combination is added dropwise to the reaction mixture.

The reaction mixture is further stirred at room temperature for around 18-24 hours. Then the reaction mixture was quenched with at least an acid preferably inorganic acid, such as HCl, H₂SO₄ and/or H₃PO₄ solution. The obtained organic layer was subsequently washed with water. The resulting solution is evaporated to the residue at reduced pressure using rotatory evaporator.

Then the product is recrystallized from at least one of tert-butyl methyl ether, tetrahydrofuran, diethyl ether, dioctyl ether, chloromethyl methyl ether, diethylene glycol dimethyl ether and/or diphenyl ether, providing 1,4:3,6-dianhydro-2,5-di-O-p-tosyl-hexitol, preferably 1,4-3,6-dianhydro-2,5-di-O-p-tosyl-D-mannitol **(2).**

**Step b) of** the invention is synthesis of 2,5-diphthalimido-2,5-dideoxy-1,4:3,6-dianhydrohexitol, preferably 2,5-diphthalimido-2,5-dideoxy-1,4:3,6-dianhydroiditol **(3)** by reacting of 1,4:3,6-dianhydro-2,5-di-O-p-tosyl-hexitol, preferably 1,4:3,6-dianhydro-2,5-di-O-p-tosyl-D- mannitol **(2)** and a phthalimide containing compound, including but not limited to potassium phthalimide, sodium phthalimide, lithium phthalimide or any of their combination. In preferred embodiments, potassium phthalimide, 1,4-3,6-dianhydro-2,5-di-O-p-tosyl-D-mannitol **(2)** and organic solvent, which can be selected from DMSO, dichloromethane, DMF Dimethylformamide, MeCN acetonitrile, THF tetrahydrofuran, ethyl acetate, acetone, nitromethane, propylene carbonate and their combination, are charged into the reactor. After stirring, the reaction mixture is cooled down. Then water is added into the reaction mixture. Then the mixture is cooled down to the room temperature and the resulting precipitate is collected by filtration. After drying, compound **3** is obtained.

**In step c),** 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride **(4)** is synthesized by reacting of 2,5-diphthalimido-2,5-dideoxy-1,4:3,6-dianhydrohexitol, preferably 2,5-diphthalimido-2,5-dideoxy-1,4:3,6-dianhydroiditol **(3)** with at least an acid, preferably a mixture of HCl and AcOH. The volume ratio of HCl/AcOH is preferably 5:1-3:1, more preferably 4:1.

Compound **3** and an acid and at least a chloride source, preferably HCl, more preferably a mixture of HCl and AcOH are put into a container such as a two-necked round-bottom flask. The acid can be selected from HCl, H₂SO₄, H₃PO₄, lactic acid, formic acid, citric acid, oxalic acid, uric acid, malic acid, tartaric acid, preferably a mixture of HCl/AcOH. The chloride source is selected from hydrochloric acid (HCl), sodium chloride (NaCl), potassium chloride (KCl), lithium chloride (LiCl), ammonium chloride (NH4Cl), tetrabutylammonium chloride (Bu4NCl) and any other chloride salts.

The stirred slight brownish suspension was then refluxed for above 10 hours. Optionally more acid can be added, and the reaction mixture can be further refluxed. After completion of the reaction, the slightly brown solution is cooled down to room temperature. Preferably, crystallization and filtration are conducted, and then the reaction mixture is evaporated and then filtered to provide compound **4.**

**Step d)** is reacting of 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride **(4)** and/with at least an alkali, and purification, preferably recrystallization to provide the 2,5-diamino-2,5-dideoxy-1,4:3,6-dianhydrohexitol **(5).** Preferably, the 2,5-diamino-2,5-dideoxy-1,4:3,6-dianhydrohexitol is a 2,5-diamino-2,5-dideoxy -1,4:3,6-dianhydroiditol.

A solution of compound **4** and an alkali, which can be selected from NaOH, KOH, Na₂CO₃, K₂CO₃, LiOH and their combination, in water is stirred at ambient temperature for ≥ 20 minutes, preferably ≥25 minutes. Then the reaction mixture is evaporated to dryness under vacuum and the residue was dissolved in organic solvent such as ethanol, formic acid, butanol, isopropanol, methanol, acetic acid or their combination. The mixture is heated and stirred. The resulting suspension is filtered, and the filtrate is subsequently evaporated to the residue. Then at least an organic solvent, which can be selected from dichloromethane, DMSO dimethyl sulfoxide, DMF dimethylformamide, MeCN acetonitrile, THF tetrahydrofuran, ethyl acetate, acetone, nitromethane, propylene carbonate or their combination, is added to dissolve the residue. The mixture is filtered and recrystallized from an organic solvent, which can be selected from tert-butyl methyl ether, tetrahydrofuran, diethyl ether, dioctyl ether, chloromethyl methyl ether, diethylene glycol dimethyl ether, diphenyl ether, to provide compound **5.**

In one of the preferred embodiments of the invention, each step of the method can be described as below. The following image shows each step of the method in synthesis of 2,5-diamino-2,5-dideoxy-1,4:3,6-dianhydrohexitol, preferably 2,5-diamino-2,5-dideoxy-1,4:3,6-dianhydroiditol **(5)** from 1,4:3,6-dianhydrohexitol , preferably isomannide **(1)** to 1,4:3,6-dianhydro-2,5-di-O-p-tosyl-hexitol, preferably 1,4:3,6-dianhydro-2,5-di-O-p-tosyl-D-mannitol **(2),** 2,5-diphthalimido-2,5-dideoxy-1,4:3,6-dianhydrohexitol, preferably 2,5-diphthalimido-2,5-dideoxy-1,4:3,6-dianhydroiditol **(3),** 2, 5-diamino-2, 5-dideoxy-1, 4: 3, 6-dianhydrohexitol dihydrochloride, preferably 2, 5-diamino-2, 5-dideoxy-1, 4: 3, 6-dianhydroiditol dihydrochloride **(4)** and then to 2,5-diamino-2,5-dideoxy-1,4:3,6-dianhydrohexitol, preferably 2,5-diamino-2,5-dideoxy-1,4:3,6-dianhydroiditol **(5).**

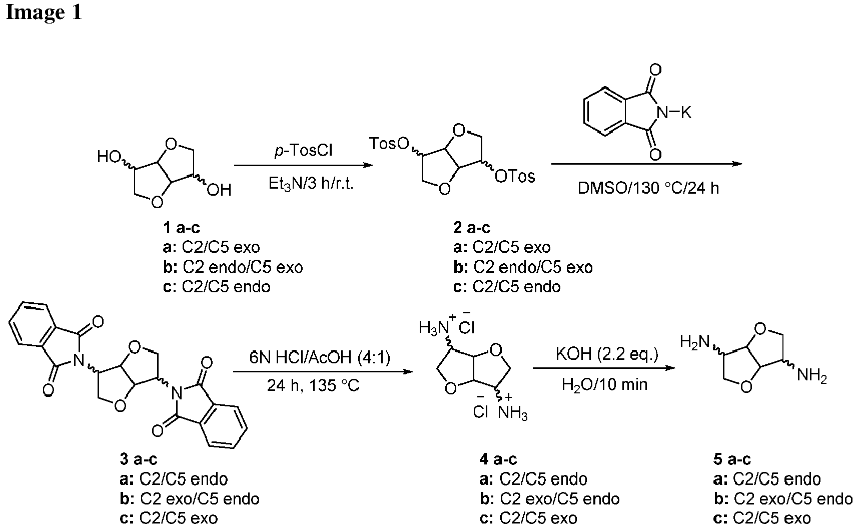

A person skilled in the art is aware that some bio-based chemicals can be an alternative or even replace certain well-known used raw materials from petro streamlines in laboratory. However, in principle, scaling up is extremely difficult and the yields are usually not possible to enable commercialization.

What is essential to the invention is that through numerous experiments, we surprisingly find that, the combination of the steps a)-d) of the method of the present invention has fulfilled not only an efficient route of producing a bio-based diaminoisoidides, but much larger scale of production with high yields.

Specifically, the procedure of the invention gives rise to the following advantages for raw materials for the preparation of isocyanates:
I. successfully achieved a much larger scale of production for bio-based diaminoisoidides;
II. significantly improved the yield of the final product of bio-based diaminoisoidides as well as the intermediates;
III. successfully reduced the use of organic solvents and mitigated the emission of TVOC and therefore more environment-friendly;
IV. using more suitable chemicals and/or process such as alkali and recrystallization to avoid application of expensive cation exchange resin, making the method more economic and hereof more attractive for industrial development;
V. enabling a big step towards commercialization of bio-based diaminoisoidides in producing isocyanates and therefore enhancing the sustainability and use of renewable feedstocks for future economy.

Thus, the method of the invention enables, by ensuring above mentioned advantages, a big step towards sustainable energy and economy.

### Examples

### Test Methods

Weight, using electronic balance (OHAUS and Techcomp) to weigh the weight of the chemicals;
Purity, according to analysis of HNMR spectra;
Molar yield of the product of the invention is calculated by molar yield = weight of substance actually generated / weight of substance theoretically generated ∗ 100%. The molar yield of the product from each step is calculated separately per aforementioned formula.

Weight of substance theoretically generated = weight of the starting substance / molecular weight of the starting substance ∗ molecular weight of the product.

### Raw Materials

Pyridine, ≥99.5%, Greagent;
Potassium phthalimide, 98%, 3A Chemicals;
p-toluene sulphonyl chloride, 99%, Adamas;
CH₂Cl₂, ≥99.5%, Greagent;
MgSO₄, ≥98%, Greagent;
G-3 filter funnel containing Celite, ≥85.0%, Greagent;
Methanol, ≥99.5%, Greagent;
Amberlyst A-26-OH, Changzhou Tehua Trading Company;
Isomannide, 97%, 3A Chemicals;
triethylamine, ≥99.0%, Greagent;
HCl solution, 36.0-38.0%, Sinopharm Chemical Reagent Co.,Ltd;
tert-butyl methyl ether, ≥99.0%, Greagent;
DMSO, ≥99.0%, Greagent;
AcOH, ≥99.5%, Sinopharm Chemical Reagent Co.,Ltd;
chromatography (TLC), Rushan Taiyang Desiccant Co.,Ltd;
ethanol, ≥99.7%, Greagent;

### Example 1 (comparative example)

A comparative example is prepared per a known method as following.

### Step 1

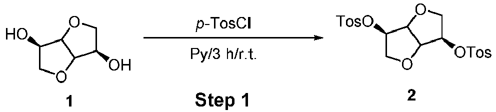

A 2-necked round-bottom flask was charged with a solution of isomannide (200.0 g, 1.37 mol) in 300 mL of pyridine under nitrogen atmosphere. The mixture was cooled to 0-5 °C. A solution of p-toluene sulphonyl chloride (522.0 g, 2.74 mol) in pyridine (1500 mL) was added dropwise over 30 min. After 3 h, the reaction mixture was placed in a refrigerator overnight. Then, the mixture was poured onto ice-water (10.0 L) and stirred for 1 h. The obtained sandy solid was subsequently grounded and washed thoroughly with water, dilute HCl (0.2 M), and finally water followed by suction-filtration. The product was recrystallized from ethanol yielding 1,4-3,6-dianhydro-2,5-di-O-p-tosyl-D-mannitol 2 as white needles (485 g, 78% yield).

### Step 2

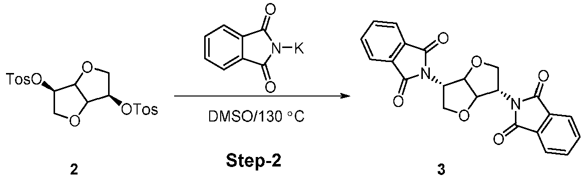

Potassium phthalimide (44.4 g, 0.24 mol) and DMSO (650 mL) was charged into the reactor under a continuous flow of nitrogen. The mixture was stirred at 130 °C (internal temperature) for 4 h. 150 mL of DMSO was distilled off under reduced pressure. The reaction mixture was cooled down to room temperature and 1,4-3,6-dianhydro-2,5-di-O-p-tosyl-D-mannitol 2 (50.0 g, 0.11 mol) was added. Again, the mixture was stirred at 130 °C (internal temperature) for 24 h. After the completion of reaction, the dark brown solution was cooled down to room temperature and poured into cold water (1 L) (pH 7/8). The resulting DMSO/water mixture was extracted with CH2Cl2 (4×500 mL). The combined CH2Cl2 layers were then subsequently washed with water (2×500 mL), dried over MgsO4 and decolorized with activated carbon (30 min at room temperature). The resulting solution was then filtered over a G-3 filter funnel containing Celite. The brown filtrate was evaporated at reduced pressure using rotatory evaporator, giving a yellow solid. The crude product was further purified by selective precipitation of 2,5-diphthalimido-2,5-dideoxy-1,4-3,6-dianhydroiditol 3 from 250 mL of chloroform (20.0 g, 44.5% yield).

### Step 3

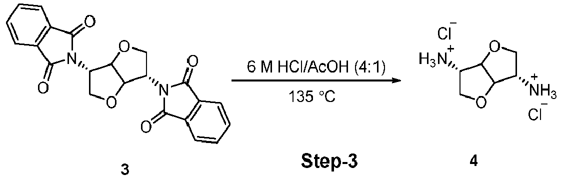

A two-necked round-bottom flask was charged with 3 (10.0 g, 24.7 mmol) and 60 mL 6 N HCl/AcOH (4:1). The stirred slight brownish suspension was then heated at 135 °C (oil bath temperature) for 24 h. After completion of the reaction, the slightly brown solution was cooled down to room temperature. During cooling crystallization of phthalic acid occurred. The resulting suspension was filtered over a G-3 filter funnel. The brownish filtrate was washed thoroughly with diethyl ether (3×100 mL) to remove residual phthalic acid, and the aqueous phase was subsequently evaporated to dryness using a rotary film evaporator yielding a brown solid. This was dissolved in methanol (50 mL) at 50 °C. Then ethanol (50 mL) was added, and the temperature was raised to distill off the methanol. Subsequently, an ethanolic HCl solution (20 mL) was added to precipitate the desired product. The resulting suspension was stirred for 10 min at 50 °C, filtered over a G-3 filter funnel and the precipitate was dried in a vacuum oven (<5 mbar, 24 h) over Sicapent yielding 2, 5-diamino-2, 5-dideoxy-1, 4: 3, 6-dianhydrosorbitol dihydrochloride 4 (3.8 g, 70.8% yield) as a slightly brown solid.

### Step 4

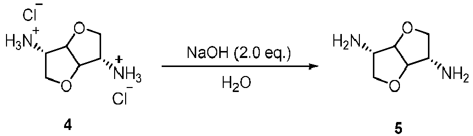

2, 5-diamino-2, 5-dideoxy-1, 4: 3, 6-dianhydrosorbitol dihydrochloride 4 (5 g, 23.0 mmol) was dissolved in water (50 mL) giving a brown solution. To this solution freshly washed Amberlyst A-26-OH (19.6 g, 24.5 mmol) was added, and the resulting suspension was sonified in an ultrasonic bath for 1 h at 30 °C. However, the pH value of the solution was ∼6-7 which means not all Cl atoms are replaced by hydroxyl groups. No final target product was obtained.

Comparative data shows no final target product was obtained through the known method.

### Example 2

Examples 2 and 3 are preferred embodiments of the invention, which can be shown in above Image 1 and the following detailed procedures.

Certain amount of isomannide (400.0 g, 2.74 mol), p-toluenesulfonyl chloride (1149 g, 6.03 mol) and 300 mL of dichloromethane were added to a 2-necked round-bottom flask. Then triethylamine (830 g, 8.20 mol) was added dropwise to the reaction mixture under stirring at 25-35 °C. The reaction mixture was further stirred at 25-35 °C for 21 h. Then the reaction mixture was quenched with 2000 mL of 2 M HCl solution. The obtained organic layer was subsequently washed with 2000 mL of H₂O. The resulting solution was evaporated to the residue at reduced pressure using rotatory evaporator. Then the product was recrystallized from tert-butyl methyl ether yielding 1,4-3,6-dianhydro-2,5-di-O-p-tosyl-D-mannitol **(2)** as white powders (1139.6 g, 91.6% yield).

Potassium phthalimide (293.2 g, 1.58 mol), 1,4-3,6-dianhydro-2,5-di-O-p-tosyl-D-mannitol **(2)** (327.0 g, 0.72mol) and DMSO (1600 mL) was charged into the reactor under a continuous flow of nitrogen. After stirring at 135 °C for 17.5 h, the reaction mixture was cooled down to 65-75 °C. Then 1600mL of H₂O was added dropwise into the reaction mixture at 65-75 °C. Then the mixture was cooled down to the room temperature and the resulting precipitate was collected by filtration. After drying, 2,5-diphthalimido-2,5-dideoxy-1,4-3,6-dianhydroiditol **(3)** was obtained as faint yellow powders (166.2 g, 57.1% yield).

A two-necked round-bottom flask was charged with compound **3** (166.2 g, 0.41 mol) and 1550 mL 6 M HCl/AcOH (4:1). The stirred slight brownish suspension was then refluxed for 12 h. The reaction progress was monitored by thin-layer chromatography (TLC) and compound **3** was not all consumed. Then 330 mL 6 M HCl/AcOH (4:1 v/v) was added and the reaction mixture was further refluxed for 18 h. After completion of the reaction, the slightly brown solution was cooled down to room temperature. During cooling crystallization of phthalic acid occurred. The resulting suspension was filtered, and the filtrate was subsequently evaporated to dryness using a rotary film evaporator yielding a brown sticky solid. Then ethanol (1600 mL) was added, the resulting suspension was stirred and filtered to collect the wet cake yielding compound **4** as an off-white solid (143.8g, 92.5% yield).

A solution of 2,5-diamino-2,5-dideoxy-1,4-3,6-dianhydroiditol dihydrochloride (10.0 g, 46.1 mmol) and NaOH (3.68 g, 92.2 mmol) in water (60.0 mL) was stirred at ambient temperature for 30 min. Then the reaction mixture was evaporated to dryness under vacuum and the residue was dissolved in 130 mL of ethanol. The mixture was heated to 50 °C and stirred for 3 h. The resulting suspension was filtered, and the filtrate was subsequently evaporated to the residue using a rotary film evaporator. Then 40 mL of dichloromethane was added to dissolve the residue. The mixture was filtered, and the filtrate was concentrated to the residue. The product was recrystallized from tert-butyl methyl ether yielding compound **5** as off-white needles (5.97 g, 89.8% yield).

### Example 3

Isomannide (50.0 g, 0.34 mol), p-toluene sulphonyl chloride (143.5 g, 0.75 mol) and 300 mL of dichloromethane were added to a 2-necked round-bottom flask. Then triethylamine (103.8 g, 1.03 mol) was added dropwise to the reaction mixture under stirring at 25-35 °C. The reaction mixture was further stirred at 25-35 °C for 14 h. Then the reaction mixture was quenched with 200 mL of 2 M HCl solution. The obtained organic layer was subsequently washed with 200 mL of H₂O. The resulting solution was evaporated to the residue at reduced pressure using rotatory evaporator. Then the product was recrystallized from tert-butyl methyl ether yielding 1,4-3,6-dianhydro-2,5-di-O-p-tosyl-D-mannitol 2 as white powders (143.8 g, 92.5% yield, purity 99wt%).

Potassium phthalimide (8.5 g, 45.6 mmol), 1,4-3,6-dianhydro-2,5-di-O-p-tosyl-D-mannitol (9.4 g, 20.7 mmol) and DMSO (50 mL) were charged into the reactor under a continuous flow of nitrogen. After stirring at 135 °C for 18.5 h, the reaction mixture was cooled down to 70-80 °C. Then 50 mL of H₂O was added dropwise into the reaction mixture at 70-80 °C. Then the mixture was cooled down to the room temperature and the resulting precipitate was collected by filtration. After drying, 2,5-diphthalimido-0.452,5-dideoxy-1,4-3,6-dianhydroiditol **(3)** was obtained as faint yellow powders (5.3 g, 63.4% yield, purity 99wt%).

A two-necked round-bottom flask was charged with 3 (3.67 g, 9.08 mmol) and 44 mL 6 M HCl/AcOH (4:1 v/v) and a slightly brown suspension was obtained. The slightly brown suspension was stirred and then refluxed for 12 h. The reaction progress was monitored by TLC. After completion of the reaction, the slightly brown suspension was cooled down to room temperature. During cooling, the slightly brown suspension was through crystallization of phthalic acid. The resulting suspension was filtered, and the filtrate was subsequently evaporated to dryness using a rotary film evaporator yielding a brown sticky solid. Then ethanol (40 mL) was added, the resulting suspension was stirred and filtered to collect the wet cake yielding compound **4** as an off-white solid (1.72g, 87.3% yield, purity 99wt%).

A solution of 2,5-diamino-2,5-dideoxy-1,4-3,6-dianhydroiditol dihydrochloride (3.0 g, 13.8 mmol) and NaOH (1.11 g, 27.8 mmol) in water (6.0 mL) were stirred at ambient temperature for 30 min. Then the reaction mixture was evaporated to dryness under vacuum and the residue was dissolved in 40 mL of ethanol. The mixture was heated to 50 °C and stirred for 3 h. The resulting suspension was filtered, and the filtrate was subsequently evaporated to the residue using a rotary film evaporator. Then 4 mL of dichloromethane was added to dissolve the residue. The mixture was filtered, and the filtrate was concentrated to the residue. The product was recrystallized from tert-butyl methyl ether yielding compound **5** as off-white needles (1.75 g, 88% yield, purity 99wt%).

As the examples show, surprisingly, when we use the method of the present invention, not only the final product is obtained, but a much larger scale of production of the diaminoisoidides is successfully fulfilled as well. Moreover, the quality, the purity and yield are significantly improved. And, much fewer organic solvents are used.

## Claims

1. A method for producing 2,5-diamino-2,5-dideoxy-1,4:3,6-dianhydrohexitol, comprising:
a). synthesis of 1,4:3,6-dianhydro-2,5-di-O-p-tosyl-hexitol by reacting of 1,4:3,6-dianhydrohexitol and at least a toluenesulfonyl containing compound;
b). synthesis of 2,5-diphthalimido-2,5-dideoxy-1,4:3,6-dianhydrohexitol by reacting of 1,4:3,6-dianhydro-2,5-di-0-p-tosyl-hexitol and at least a phthalimide containing compound;
c). synthesis of 2, 5-diamino-2, 5-dideoxy-1, 4: 3, 6-dianhydrohexitol dihydrochloride by reacting of 2,5-diphthalimido-2,5-dideoxy-1,4:3,6-dianhydrohexitol with at least an acid and at least a chloride source, preferably HCl, more preferably a mixture of HCl and AcOH;
d). reacting of 2, 5-diamino-2,5-dideoxy-1, 4: 3, 6-dianhydrohexitol dihydrochloride and at least an alkali, preferably NaOH, and purification, preferably recrystallization, to provide the 2,5-diamino-2,5-dideoxy -1,4:3 ,6-dianhydrohexitol.

2. The method according to claim 1, wherein the 2,5-diamino-2,5-dideoxy-1,4:3,6-dianhydrohexitol has a structure of: 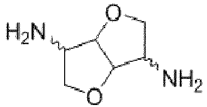
**5 a-c**
**a:** C2/C5 endo
**b:** C2 exo/C5 endo
**c:** C2/C5 exo ;
and, preferably a structure of: 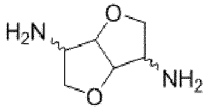
**5 a**
**a:** C2/C5 endo

3. The method according to any of the preceding claims, wherein the toluenesulfonyl containing compound is selected from p-toluenesulfonyl chloride, p-toluenesulfonyl hydrazide, p-toluenesulfonyl isocyanate, p-toluenesulfonyl azide, p-toluenesulfonyl cyanide, m-toluenesulfonyl chloride and o-toluenesulfonyl chloride.

4. The method according to any of the preceding claims, wherein there is no cation exchange resin used in the step d).

5. The method according to any of the preceding claims, wherein there is no organic solvent applied in step b).

6. The method according to any of the preceding claims, wherein the purification in step d) is selected from recrystallization.

7. The method according to any of the preceding claims, wherein the amount of 1,4:3,6-dianhydrohexitol in step a) is ≥300g, preferably ≥320g, and more preferably ≥380g.

8. The method according to any of the preceding claims, wherein the molar yield of 1,4:3,6-dianhydro-2,5-di-O-p-tosyl-hexitol in step a) is ≥50%, preferably ≥80%, more preferably ≥90%.

9. The method according to any of the preceding claims, wherein the molar yield of the 2,5-diphthalimido-2,5-dideoxy-1,4:3,6-dianhydrohexitol from step b) is ≥50%, preferably ≥60%.

10. The method according to any of the preceding claims, wherein the phthalimide containing compound is selected from potassium phthalimide, sodium phthalimide, lithium phthalimide or any of their combination.

11. The method according to any of the preceding claims, wherein the molar yield of the 2, 5-diamino-2, 5-dideoxy-1, 4: 3, 6-dianhydrohexitol dihydrochloride in step c) is ≥80%, preferably ≥90%.

12. The method according to any of the preceding claims, wherein the molar yield of the 2,5-diamino-2,5-dideoxy-1,4:3,6-dianhydrohexitol is ≥80%, preferably ≥90%.

13. 2,5-diamino-2,5-dideoxy-1,4:3,6-dianhydrohexitol prepared by the method according to any of the claims 1-12.

14. The 2,5-diamino-2,5-dideoxy-1,4:3,6-dianhydrohexitol according to claim 13, the purity of the 2,5-diamino-2,5-dideoxy-1,4:3,6-dianhydroiditol is ≥98%, preferably ≥99%.

15. Use of the 2,5-diamino-2,5-dideoxy-1,4:3,6-dianhydrohexitol prepared by the method according to any of the claims 1-12 in producing isocyanates.
